# EUROPÄISCHE PATENTSCHRIFT

(11) **EP 0 923 341 B1**
(45) Veröffentlichungstag und Bekanntmachung des Hinweises auf die Patenterteilung: **13.02.2002**
(21) Anmeldenummer: 97938748.7
(22) Anmeldetag: 06.08.1997
(51) Int. Cl.: A61B 5/117, A61B 5/00, G01K 7/00

(54) **VERFAHREN UND ANORDNUNG ZUR IDENTIFIKATION VON PERSONEN**
PROCESS AND SYSTEM FOR IDENTIFICATION OF PERSONS
PROCEDE ET DISPOSITIF POUR L'IDENTIFICATION DE PERSONNES

(30) Priorität: 28.08.1996 DE 19634849
(43) Veröffentlichungstag der Anmeldung: 23.06.1999
(73) Patentinhaber: Infineon Technologies AG, 81669 München (DE)
(72) Erfinder: BULST, Wolf-Eckhart, D-81739 München (DE); WERSING, Wolfram, D-81667 München (DE); BRUCHHAUS, Rainer, D-80997 München (DE)
(74) Vertreter: Fischer, Volker, Dipl.-Ing.
(86) Internationale Anmeldenummer: DE9701659
(87) Internationale Veröffentlichungsnummer: WO9808438

(56) Entgegenhaltungen:
- EP-A- 0 071 269
- WO-A-80/01514
- FR-A- 2 736 179
- US-A- 4 428 382

## Beschreibung

In praktisch allen Bereichen des täglichen Lebens geht heute die Tendenz dahin, persönliche Bedürfnisse jederzeit und überall so einfach wie möglich befriedigen zu können. Zu diesem Zweck müssen Personen in der Regel von Menschen oder Geräten sicher identifiziert und als berechtigt akzeptiert werden können.

In der Praxis ist dies heute noch aufwendig und umständlich, indem z.B. Ausweise ggf. mit Lichtbild vorgezeigt und überprüft, Unterschriften geleistet oder Schlüssel, Karten und Codenummern benutzt werden müssen. Bei allen diesen Identfikations-Hilfsmitteln besteht jedoch die Gefahr des Abhandenkommmens, der widerrechtlichen Benutzung oder Fälschung, des Verlustes, Verlegens oder Vergessens. Es werden daher in neuerer Zeit Versuche unternommen, derartige Identifizierungsmittel durch körpereigene Identifizierungsmerkmale zu ersetzen, die leicht und sicher identifizierbar sind. Eine schnelle und sichere maschinelle Identifizierung von Personen mittels körpereigener Identifikationsmerkmale wurde bei den heute vorhandenen Möglichkeiten der Computertechnik und Vernetzung einen weltweiten und automatischen Individual-Service möglich machen.

Zu diesem Zweck sind bereits Verfahren und Anordnungen zur Bestimmung der Echtheit und/oder der Identifikation von Personen bekannt geworden, die auf der Ausnutzung von Ultraschall beruhen. Dazu wird beispielsweise auf die DE-OS 36 10 397 und 42 22 387 sowie auf die EP-A 0 402 779 verwiesen. Diese Verfahren beruhen auf der Erkennung Charakteristika von lebendem Gewebe speziell in einer Fingerkuppe einer zu identifizierenden Person und sind daher auch juristisch zulässig.

Die menschliche Epidermis bzw. Oberhaupt, also auch von Fingerkuppen besteht aus einer Hornschicht, einer Keimschicht und einer Baselschicht. Für Fingeroberflächen ist eine Leistenstruktur, d. h. eine Folge von Erhabenheiten bzw. Leisten und zwischen diesen befindlichen Furchen typisch, wie sie sich in einem Fingerabdruck abbildet. Diese Leistenstruktur wiederholt sich in einer tiefer liegenden Hautschicht, so daß sich diese Hautschicht ebenfalls zur Selektion von personenspezifischen Merkmalen eignet. Ein Vorteil der Detektion der tieferen Hautschichten ist eine höhere Fälschungssicherheit und eine gleichbleibende Erkennung bei einer oberflächlichen Verletzung der Haut.

Verfahren und Anordnungen der oben genannten Art beruhen auf dem Aussenden von Ultraschall-Abfragesignalen, deren Reflexion an den vorgenannten Hautstrukturen, dem Empfang dieser Ultraschallreflexionen, deren Umwandlung in elektrische Signale und deren Auswertung zur Personenidentifizierung. Solche Verfahren und Anordnungen sind aber insofern gerätetechnisch aufwendig, als sie noch gesonderte Ultraschallquellen und ggf. zusätzlich sogar auch Lichtquellen benötigen. Zu einer Ausführungsform mit Ultraschallquelle und Lichtquelle sei beispielsweise auf die vorstehend bereits genannte DE-OS 42 22 397 verwiesen.

Aus der US 4 429 413 ist ein Sensor zur Bestimmung eines Fingerabdrucks bekannt, bei dem dieser mittels einer piezo- oder pyroelektrischen Schicht in ein Ladungsmuster umgesetzt wird, welches den Arbeitspukt von darunter zu einem Array angeordneten Transistoren beeinflußt.

Der vorliegenden Erfindung liegt die Aufgabe zugrunde, ein Verfahren zur Identifizierung von Personen über deren Epithelstruktur anzugeben, das eine einfache und direkte Messung erlaubt und ohne zusätzliche Signalquellen - wie etwa die genannten Ultraschallquellen und/oder Lichtquellen - auskommt.

Diese Aufgabe wird bei einem Verfahren nach dem Patentanspruch 1 gelöst. Zur Durchführung dieses Verfahrens wird die Anordnung nach Patentanspruch 4 verwendet. Weiterbildungen der Erfindung sind Gegenstand von Unteransprüchen.

Die Erfindung wird nachfolgend anhand eines Ausführungsbeispiels gemäß den Figuren der Zeichnung näher erläutert. Es zeigt:
Figur 1 eine schematische Darstellung eines pyroelektrischen Infrarot-Detektor-Arrays zur Durchführung des erfindungsgemäßen Verfahrens; und
Figur 2 eine schematische geschnittene Darstellung des Detektor-Arrays nach Figur 1 zur Erläuterung des Aufbaus einer pyroelektrischen Detektorzelle.

Im Grundsatz macht sich die Erfindung von der Tatsache zunutze, daß lebendes Gewebe von wärmetransportierenden Körperflüssigkeiten, wie beispielsweise Blut, durchströmt wird und daß sich Hautstrukturen insbesondere auch in unter der Oberfläche liegenden Hautschichten in einer entsprechenden Temperaturverteilung widerspiegeln. Erfindungsgemäß werden solche Temperaturverteilungen zu Identifizierungszwecken detektiert und ausgewertet. Die Temperaturverteilungen sind individuell eindeutig zuzuordnen, weil die in tiefer liegenden Hautschichten befindlichen Strukturen unabhängig von Veränderungen an der Hautoberfläche unveränderbar bleiben.

Die Identifizierungssicherheit wird erfindungsgemäß dadurch erhöht, daß die Temperaturverteilung zeitlich getaktet gemessen wird, wodurch ein Wärmeflußdiagramm entsteht und zur Auswertung herangezogen werden kann.

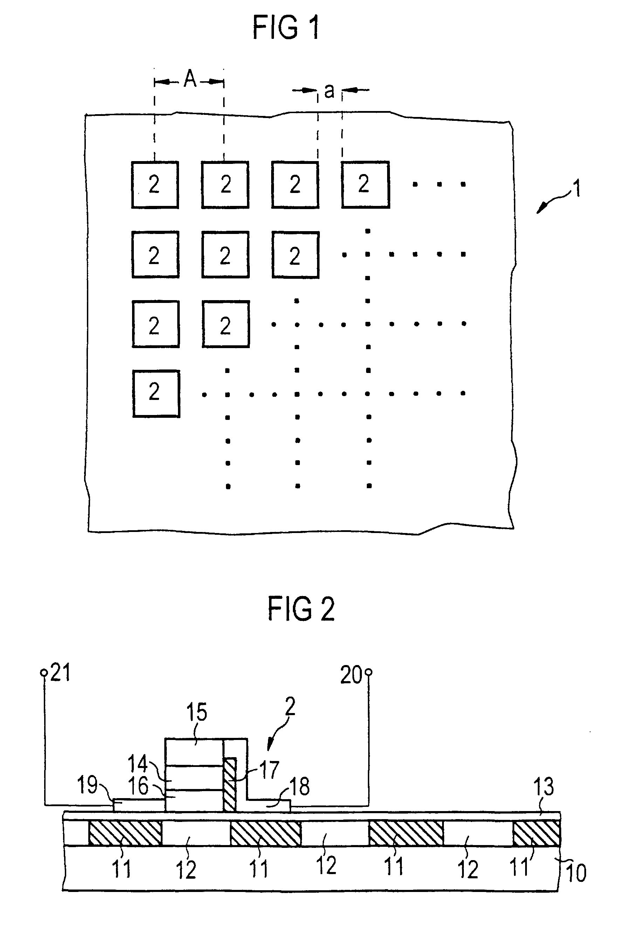

Erfindungsgemäß können diese Temperaturverteilungen durch ein pyroelektrisches Infrarot-Detektor-Array detektiert werden. Ein derartiges Detektorarray ist in Figur 1 schematisch dargestellt und mit 1 bezeichnet. Dieses Detektor-Array 1 enthält eine Vielzahl von in Zeilen und Spalten angeordneten pyroelektrischen Detektorzellen 2. Um die Temperaturverteilungen der obengenannten Art in lebendem Gewebe erfassen zu können, besitzt das Detektor-Array 1 entsprechende Zellengrößen und Zellenabmessungen. Die Temperaturverteilung in einer Fingerkuppe kann mittels eines derartigen Detektor-Arrays entweder kontaktlos oder in Kontakt mit der Fingerkuppe gemessen werden. Zur Identifizierung wird also ein Finger entweder in die Nähe des Detektor-Arrays gebracht oder auf dieses aufgelegt.

Anhand von Figur 2 wird eine Möglichkeit des prinzipiellen Aufbaus eines Detektor-Arrays nach Figur 1 beschrieben. Auf einem Substrat 10, das beispielsweise aus Silizium besteht, ist ein Muster aus Inseln 11 aus isolierendem Material, beispielsweise Siliziumdioxid mit zwischen diesen befindlichen Hohlräumen 12 vorgesehen. Dieses Muster von isolierenden Inseln 11 und Hohlräumen 12 ist mit einer Isolationsschicht 13, beispielsweise aus Siliziumnitrid abgedeckt.

Über den Hohlräumen 12 sind pyroelektrische Zellen vorgesehen, die durch ein pyroelektrisches Material 14, beispielsweise Bleicirkonat-Titanat zwischen einer oberen Elektrode und einer unteren Elektrode 16 gebildet sind. Die Elektroden 15, 16 sind mit Kontaktierungen 18, 19 vorgesehen, wobei zur Isolation der Kontaktierung 18 für die obere Elektrode 15 gegen das pyroelektrische Material 14 und die untere Elektrode 16 ein Isolator 17 beispielsweise aus Aluminiumoxid oder Siliziumoxid vorgesehen ist. Über Außenanschlüsse 20 und 21 ist die pyroelektrische Zelle elektrisch zugänglich. Die Zelle 2 der in Figur 2 dargestellten Art entspricht den Zellen 2 nach Figur 1. Die Hohlräume 12 zwischen den Inseln 11 aus isolierendem Material sind evakuiert, um eine Wärmeisolation zwischen dem Substrat 10 und der pyroelektrischen Zelle 2 zu gewährleisten.

## Patentansprüche

1. Verfahren zur Identifizierung von Personen durch Erkennung von Charakteristika von lebendem Gewebe der zu identifizierenden Person, bei dem die Temperaturverteilung in der Hautstruktur in der Fingerkuppe der zu identifizierenden Person mit einem Pyrodetektorarray detektiert wird, welches pyroelektrische Zellen umfaßt, in denen eine pyroelektrische Schicht zwischen einer oberen und einer unteren Elektrode angeordnet ist,wobei die Temperaturverteilung zur Ermittlung eines Wärmeflußdiagramms zeitlich getaktet detektiert wird.

2. Verfahren nach Anspruch 1,
bei dem die Temperaturverteilung in der Fingerkuppe kontaktlos detektiert wird.

3. Verfahren nach Anspruch 1,
bei dem die Temperaturverteilung im Kontakt mit der Fingerkuppe detektiert wird.

4. Anordnung zur Durchführung des Verfahrens nach einem der Ansprüche 1 bis 3,
mit einem pyroelektrisches Infrarotdetektor-Array (1, 2) zur Temperaturerfassung, welches pyroelektrische Zellen umfaßt, in denen eine pyroelektrische Schicht zwischen einer oberen und einer unteren Elektrode angeordnet ist und mit Mitteln zur zeitlich getakteten Detektion der Temperaturverteilung.

## Claims

1. Method for the identification of persons by the recognition of characteristics of living tissue of the person to be identified, in which the temperature distribution in the skin structure in the finger-tip of the person to be identified is detected by means of a pyro-detector array comprising pyroelectric cells, in which a pyroelectric layer is arranged between an upper and a lower electrode, the temperature distribution being detected in a clocked manner for the purpose of determining a heat flow chart.

2. Method according to Claim 1, in which the temperature distribution in the finger-tip is detected contactlessly.

3. Method according to Claim 1, in which the temperature distribution is detected in contact with the finger-tip.

4. Arrangement for carrying out the method according to one of Claims 1 to 3, with a pyroelectric infrared detector array (1, 2) for the recording of temperature, which comprises pyroelectric cells, in which a pyroelectric layer is arranged between an upper and a lower electrode and with means for detecting the temperature distribution in a clocked manner.

## Revendications

1. Procédé pour l'identification de personnes par reconnaissance de caractéristiques d'un tissu vivant de la personne à identifier, dans lequel la répartition de température dans la structure cutanée au bout d'un doigt de la personne à identifier est détectée par un réseau de détecteurs pyro-électriques qui comporte des cellules pyro-électriques dans lesquelles une couche pyro-électrique est montée entre une électrode supérieure et une électrode inférieure, la répartition de température étant détectée de manière cadencée dans le temps afin de déterminer un diagramme de flux thermique.

2. Procédé suivant la revendication 1, dans lequel la répartition de température au bout d'un doigt est détectée sans contact.

3. Procédé suivant la revendication 1, dans lequel la répartition de température est détectée en contact avec le bout du doigt.

4. Dispositif pour la mise en oeuvre du procédé suivant l'une des revendications 1 à 3, comportant un réseau (1, 2) de détecteurs infrarouges pyro-électriques pour la détection de température, qui comprend des cellules pyro-électriques dans lesquelles une couche pyro-électrique est disposée entre une électrode supérieure et une électrode inférieure et comportant des moyens pour détecter de manière cadencée dans le temps la répartition de température.
